(19) Europäisches Patentamt
European Patent Office
Office européen des brevets

(11) Publication number: 0 142 920
A2

(12) # EUROPEAN PATENT APPLICATION

(21) Application number: 84306433.8

(22) Date of filing: 20.09.84

(51) Int. Cl.⁴: C 07 D 215/20

(30) Priority: 20.09.83 US 535522

(43) Date of publication of application: 29.05.85 Bulletin 85/22

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Titus, Robert Daniel
3818 Spann Avenue
Indianapolis Indiana 46203(US)

(72) Inventor: Bach, Nicholas James
7215 South Meridian
Indianapolis Indiana 46217(US)

(74) Representative: Tapping, Kenneth George et al,
Lilly Industries Limited Patent Department Erl Wood Manor
Windlesham Surrey, GU20 6PH(GB)

(54) **6-Oxodecahydroquinolines and process for their preparation.**

(57) Trans-(±)-6-hydroxy(or oxo)decahydroquinoline and its trans-(−)-stereoisomer, useful as intermediates, are disclosed herein. The compounds are prepared by cleaving the corresponding 1-cyano derivative, by oxidizing the corresponding 6-hydroxy derivative, by reducing the corresponding 6-oxo derivative, or by cleaving the corresponding 6-ketal derivative.

EP 0 142 920 A2

# IMPROVEMENTS IN AND RELATING TO 6-OXODECAHYDROQUINOLINES

This invention concerns a class of novel 6-oxodecahydroquinolines, which are useful intermediates in preparing pharmaceuticals.

In preparing octahydropyrazolo[3,4-g]quinolines, United States Patent 4,198,415, or octahydro-2H-pyrrolo[3,4-g]quinolines, United States Patent 4,235,909, an intermediate, trans($\pm$)-1-alkyl-6-oxo-decahydroquinoline of the following structure (I) is used

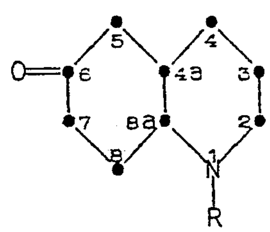

(I)

where R is $C_1$-$C_3$ alkyl or benzyl. Reaction of this compound with dimethylformamide dimethylacetal forms a 6-oxo-7-dimethylaminomethylenedecahydroquinoline (II)

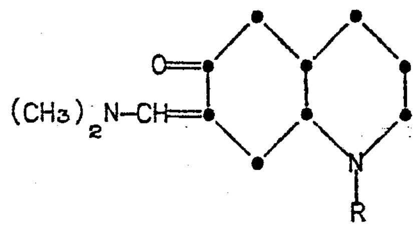

(II)

Reaction with an alkylformate yields a 7-formyl-6-oxo-decahydroquinoline (III).

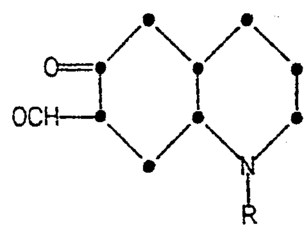

(III)

Further reaction of (II) or (III) with hydrazine or potassium glycinate yields the trans(±)-5-alkyl-octahydro-1H(and 2H)pyrazolo[3,4-g]quinoline of United States Patent 4,198,415 or the trans-(±)-5-alkyloctahydro-2H-pyrrolo[3,4-g]quinoline of United States Patent 4,235,909, wherein R is lower alkyl or benzyl in either patent.

The same intermediates (II) and (III) react with guanidine or N-methyl guanidine or N,N-dimethylguanidine to yield the trans-(±)-2-amino(methylamino, dimethylamino)-6-alkyl-5,5a,6,7,8,9,9a,10-octahydro-

pyrimido[4,5-g]quinolines disclosed in our copending application 84305684.7.

Identical chemistry can be carried out on the separated isomers of formula (I) particularly the trans(-) (4aR,8aR) isomer (IV)

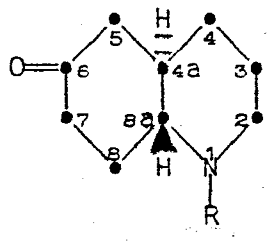

(IV)

In the pyrazolo[3,4-g]quinoline of U.S. Patent 4,198,415, the trans(-)-isomer has the prolactin inhibiting and anti-Parksonian activity of the racemate and is particularly useful in hypertension--see Hahn *et al.*, *J. Pharm. Exper. Therap.*, *224*, 206 (1983). The same situation is apparently present with the 6-alkyloctahydropyrimido[4,5-g]quinolines; i.e., the trans(-)-isomer has the great majority, if not the entire, dopamine agonist activity of the trans-(±)-racemate. The trans-(-)-isomers can be prepared by resolution of the final product--see U.K. Patent publication 2,129,423 for the resolution of trans-(±)-5-n-propyl-4,4a,5,6,7,-8,8a,9-octahydro-1H(and 2H)pyrazolo[3,4-g]quinoline. Alternatively, the trans(±)-1-alkyl or 1-benzyl-6-oxodecahydroquinoline (I) can be resolved--see U.K. Patent publication 2,129,422 for the preparation of trans-4aR-1-n-propyl-6-oxodecahydroquinoline--and the

resolved compound transformed to an octahydropyrazolo (or pyrrolo)[3,4-g]quinoline or an octahydropyrimido-[4,5-g]quinoline by the above procedures.

However, a synthetic problem exists for the preparation of final products of any of the three series in which the quinoline nitrogen is to be substituted with allyl. The method available for the synthesis of formula (I) (United States Patent 4,198,415, for example) involve hydrogenations, which could transform an allyl group to an n-propyl group. In the octahydropyrazolo-[3,4-g]quinolines or octahydropyrrolo[3,4-g]quinolines, the alkyl or benzyl group of the 1-substituted-6-oxo-decahydroquinoline can be removed by treatment with cyanogen bromide or ethyl chloroformate followed by treatment with cyanogen bromide or ethyl chloroformate followed by the synthetic procedure of Example 1 of United States Patent 4,198,315 where a 1-benzyl-6-hydroxydecahydroquinoline is transformed to 1-cyano-6-oxodecahydroquinoline (V)

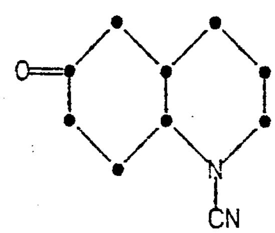

(V)

which is in turn used to prepare trans-(±)-5-cyano-4,4a,5,6,7,8,8a,9-octahydro-1H(and 2H)pyrazolo[3,4-g]-quinoline. The cyano group is then removed with Zn in acetic acid to yield the secondary amine (VI)

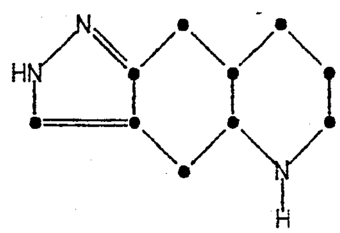

(VI)

which can be alkylated or allylated as desired.

However, since the two-ring intermediate (V) can be used to prepare at least two different three-ring compounds; i.e., 5-cyano-octahydro-1H(and 2H)pyrazolo-[3,4-g]quinolines and 2-amino-6-cyano-octahydropyrimido-[4,5-g]quinoline, either of which can be allylated after removal of the CN group, it might be preferable to form the N-allyl derivative at an earlier step in the respective synthetic procedures. In fact, because of the difference in ease of alkylating or allylating an octahydropyrazoloquinoline versus an octahydropyrimido-quinoline, it may be virtually mandatory in the second instance to carry out such an alkylation or allylation reaction prior to ring closure with guanidine. The problem is that alkylation or allylation of a secondary amine (like the quinoline ring nitrogen of the octa-hydropyrimido[4,5-g]quinoline) may be accompanied by alkylation or allylation of the primary 2-amino or secondary 2-methylamino group of the pyrimidine ring,

not to mention quaternary salt formation with the pyrimidine ring nitrogens, for example, by the alkylating moiety.

The compounds of formula (I) when R is benzoyl are disclosed by Johnson et al., J. Org. Chem., 33, 3207 (1968). Microbiological oxidation of N-benzoyl trans-(±)-decahydroquinoline provided a trans-(±)-1-benzoyl-6-hydroxydecahydroquinoline. In determining the position and configuration of the 6-hydroxyl, the trans-(+) derivative was transformed to trans-(+)-6-hydroxy(or oxo)decahydroquinoline. Neither the trans-(±)-racemate or the trans-(-)-enantiomer were prepared.

This invention provides processes and intermediates which avoid the drawbacks associated with the processes and intermediates of the prior art for preparing useful dopamine agonists containing an N-allyl group.

This invention provides trans-dl-decahydroquinoline of structure (VII) and the trans-ℓ-enantiomer thereof, structure (VIII),

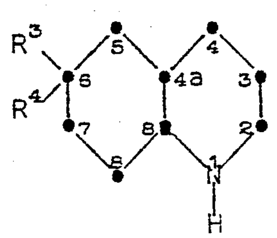

(VII)

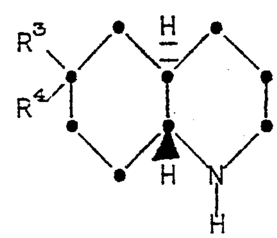

(VIII)

wherein, when taken singly, one of $R^3$ and $R^4$ is H and the other is OH, and when taken together, $R^3$ and $R^4$ form an oxo group (O=); or a pharmaceutically-acceptable acid addition salt thereof.

The compounds of formula (VII) or (VIII) are prepared by:

(a) cleaving a trans-(dl)-compound of the formula

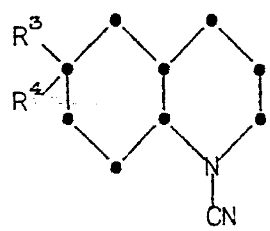

(XI)

or its trans-(-)-enantiomer of the formula

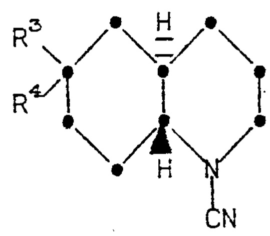

(XII)

wherein $R^3$ and $R^4$ are defined as before, by conventional methods; or

(b) oxidizing a compound of formula (VII) or (VIII) in which one of $R^3$ and $R^4$ is H and the other is OH with an oxidizing agent to provide the compounds of

formula (VII) or (VIII) in which $R^3$ and $R^4$ are taken together to form an oxo group; or

(c) reducing a compound of formula (VII) or (VIII) as defined before in which $R^3$ and $R^4$ are taken together to form an oxo group with a reducing agent to provide the compounds of formula (VII) or (VIII) as defined above in which one of $R^3$ and $R^4$ is H and the other OH; or

(d) cleaving a trans-(±)-6-ketal compound of the formula

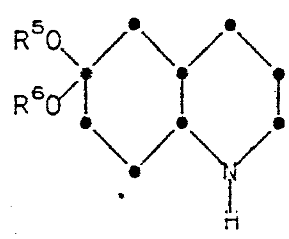

(XIII)

or its trans-(-)-enantiomer of the formula

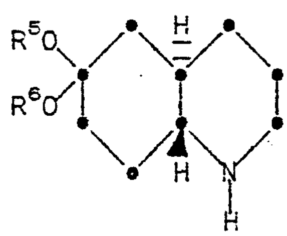

(XIV)

wherein $R^5$ and $R^6$ are each $C_1$-$C_4$ alkyl or when taken together are $-CH_2-C(CH_3)_2-CH_2-$ or $-(CH_2)_n-$ where n is 2, 3 or 4, with formic acid or an aqueous acid to provide the compounds of formula (VII) or (VIII) as defined before

in which $R^3$ and $R^4$ are taken together to form an oxo group; and

optionally followed by resolving a compound of formula (VII) to provide the corresponding trans-(-)-enantiomer; and

optionally salifying a compound of formula (VII) or (VIII) by conventional methods.

Pharmaceutically-acceptable acid addition salts of the compounds of formula (VII) or (VIII) include salts derived from non-toxic inorganic acids such as: hydrochloric acid, nitric acid, phosphoric acid, sulfuric acid, hydrobromic acid, hydriodic acid, phosphorous acid and the like, as well as salts derived from non-toxic organic acids such as aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate,

phenylbutyrate, citrate, lactate, beta-hydroxybutyrate, glycollate, malate, tartrate, methanesulfonate, propanesulfonate, naphthalene-1-sulfonate, naphthalene-2-sulfonate and the like salts.

Compounds of formula (VII) have two asymmetric centers at 4a and 8a. This structure represents 4-stereoisomers occurring as two diastereoisomeric pairs or racemates, a cis-racemate (both 4a and 8a hydrogens on the same side of the plane of the isoquinoline ring) and a trans-racemate (in which the bridgehead hydrogens at 4a and 8a are on opposite sides of the plane of the isoquinoline ring). This invention includes the trans-(±)-racemate (VII) and the trans-(-) (4aR,8aR) stereoisomer (VIII) component.

Intermediates of formula (VIII) are particularly valuable because drugs derived from them, which carry $C_1$-$C_3$ alkyl or an allyl group, contain the greater part, if not all, of the dopaminergic activity of the racemate. [The racemate (VII) can be named as a trans-(±) molecular compound or as a trans-dl-molecular compound. The stereoisomer (VIII) can be named as a trans-(-) compound, as a trans-ℓ-compound, as a 4aR,8aR derivative or as a 4aα,8aβ derivative.]

Compounds represented by formulae (VII) and (VIII) are prepared by N-dealkylating a compound of the formula (IX) or (X)

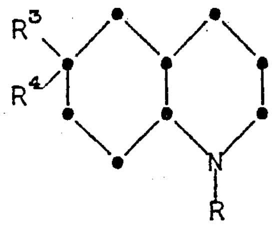
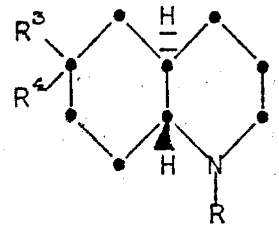

(IX) (X)

where R, $R^3$ and $R^4$ have their previous meaning, with cyanogen bromide (CN-Br) in an inert mutual solvent such as a chlorinated hydrocarbon.

The resulting 1-cyano compounds have the formula (XI) or (XII)

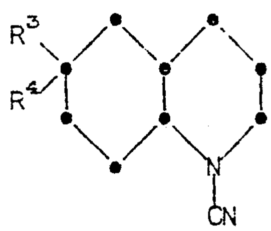
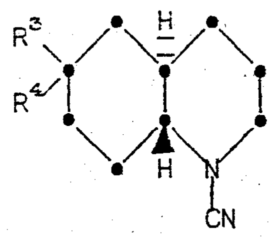

(XI) (XII)

wherein $R^3$ and $R^4$ have their previous meaning. The CN group is then removed, as by reduction with zinc and an acid, such as acetic acid, or zinc dust and HCl, or simply by treatment with acid, such as a strong mineral acid, e.g., HCl or $H_2SO_4$, or by hydrogenolysis by standard methods, for example, hydrogen and a palladium-

on-carbon or platinum-on-carbon catalyst, to yield the compounds of formulae (VII) and (VIII).

Additionally, compounds of formula (VII) or (VIII) wherein $R^3$ and $R^4$ together form an oxo group, can be prepared from compounds in which one of $R^3$ and $R^4$ is H and the other is OH by oxidation, preferably with chromic acid, or chromic anhydride in dilute $H_2SO_4$ (Jones Reagent), or pyridine hydrochloride:$CrO_3$ (Sarett's Reagent). Similarly, trans-(±)-1-cyano-6-hydroxydecahydroquinoline and the trans-(-)-stereoisomer, can be so oxidized prior to removal of the CN group.

Also, the compounds of formula (VII) or (VIII) wherein $R^3$ and $R^4$ are taken together to form an oxo group can be reduced with a reducing agent to provide the compounds of formula (VII) or (VIII) wherein one of $R^3$ and $R^4$ is H and the other is OH. Examples of suitable reducing agents are sodium borohydride, $LiBH_4$, $LiAlH_4$, $NaCNBH_3$, modified aluminum hydrides and other conventional reducing agents. Suitable solvents are $C_1$-$C_4$ alkanol such as methanol or ethanol, ethers, THF, dioxane and other solvents suitable for use with the listed reducing agents. The preferred temperature is about -5°C, to reflux, especially 0°C.

The compounds of formula (VII) or (VIII) wherein $R^3$ and $R^4$ are taken together to form an oxo group can also be prepared by cleaving a trans-(±)-6-ketal compound of the formula

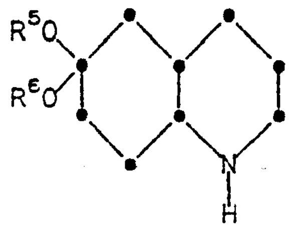

(XIII)

or its trans-(-)-enantiomer of the formula

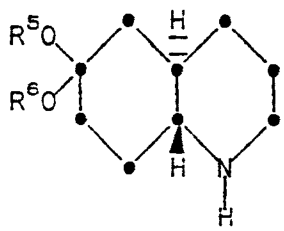

(XIV)

wherein $R^5$ and $R^6$ are each $C_1$-$C_4$ alkyl or when taken together are $-CH_2-C(CH_3)_2-CH_2-$ or $-(CH_2)_n-$ where n is 2, 3 or 4, with formic acid or an aqueous acid. Suitable aqueous acids are acetic acid, mineral acids such as hydrochloric acid, hydrobromic acid, sulfuric acid and phosphoric acid, and sulfonic acids such as p-toluenesulfonic acid, benzenesulfonic acid and methanesulfonic acid. The reaction is run at a temperature from room temperature to reflux.

The 6-ketal derivative of formula (XIII) or (XIV) above are prepared by conventional methods. One method is reaction of the 6-oxo compound with ethylene glycol in toluene with p-toluenesulfonic acid at reflux temperature. The N-substituted compounds, e.g., CN,

$C_1$-$C_3$ alkyl, having the 6-ketal present can be cleaved at the 1-position to form the 1-unsubstituted compounds by hydrogenolysis as described above. The 6-ketal group is then removed as described before to provide the 6-oxo compounds of formula (VII) or (VIII).

The compounds of formula (VII) can be resolved into the trans-(-)-enantiomer of formula (VIII) by conventional methods. For example, by recrystallization of the diastereomeric salts formed between the trans-(±) compounds and an optically active acid or by derivatizing the 1-unsubstituted compound with, for example, an amide, and separating the diastereomers.

This invention is further illustrated by the following specific examples

## Example 1

Preparation of trans-(dl)-6-oxodecahydroquinoline from the 1-benzyl derivative

Fifty-three grams of trans-dl-1-benzyl-6-hydroxydecahydroquinoline were dissolved in 1.5 l. of methylene dichloride and the solution cooled to about 0°C. in an ice-water bath. Fifty grams of cyanogen bromide were added and the resulting mixture stirred at room temperature for 15 hours. The reaction mixture was washed successively with 1N aqueous hydrochloric acid and water, and was then dried. Evaporation of the solvent yielded a residue containing trans-dl-1-cyano-6-hydroxydecahydroquinoline formed in the above reaction. The residue was dissolved in chloroform and the chloroform solution chromatographed over 300 g. of Florisil using chloroform containing increasing amounts (0-2%) of methanol as the eluant. Fractions shown by TLC to contain the desired cyano compound were combined and the solvent removed from the combined fraction by evaporation in vacuo. Trans-dl-1-cyano-6-hydroxydecahydroquinoline thus prepared weighed 22.5 g.

Twenty-two and five tenths grams of trans-dl-1-cyano-6-hydroxydecahydroquinoline were dissolved in 1200 ml. of methylene dichloride. Thirty-three grams of pyridine hydrochloride:chromium trioxide (Sarett's Reagent) were added. The reaction mixture was stirred at room temperature under nitrogen for about 6 hours, and was then filtered. The filtrate was concentrated in vacuo and the concentrate chromatographed over 300 g. of Florisil using chloroform containing 1% methanol as the eluant. Fractions shown by TLC to contain trans-dl-1-cyano-6-oxodecahydroquinoline formed in the above reaction were combined and the combined fractions evaporated to dryness in vacuo. Recrystallization of the resulting residue from an ether-chloroform solvent mixture yielded trans-dl-1-cyano-6-oxodecahydroquinoline melting at 86-8° C.; yield = 18.9 g.

Analysis Calculated: C, 67.39; H, 7.92; N, 15.71
Found: C, 67.15; H, 7.75; N, 15.46.

A reaction mixture was prepared by dissolving 2.2 g. of trans-(±)-1-cyano-6-oxodecahydroquinoline in about 50 ml. of 6N hydrochloric acid for about one hour. The volatile constituents were removed in vacuo and the

resulting residue treated with saturated aqueous sodium bicarbonate. Trans-(±)-6-oxodecahydroquinoline, being insoluble in the basic mixture, separated and was extracted by contacting the alkaline layer with several equal-volume portions of a 3:1 chloroform/isopropanol solvent mixture. The organic extracts were combined, the combined extracts washed with saturated aqueous sodium chloride and then dried. Evaporation of the solvent yielded 1.45 g. (77%) of an off-white solid consisting of trans-(±)-6-oxodecahydroquinoline. The compound was converted to the hydrochloride salt by standard procedures. The salt melted above 235°C. after crystallization from a methanol/ether solvent mixture.

Analysis Calculated: C, 56.99; H, 8.50; N, 7.38
Found: C, 57.18; H, 8.48; N, 7.17

Example 2

Preparation of trans-(±)-6-oxodecahydroquinoline and of trans-(-)-6-oxodecahydroquinoline from the 1-n-propyl derivative

About 2.0 g. of trans-(±)-1-n-propyl-6-oxodecahydroquinoline were dissolved in 35 ml. of methylene dichloride. Two and six tenths grams of cyanogen bromide were added and the mixture stirred at ambient temperature for about 2.5 hours. TLC in 2:1 THF/hexane indicated that the starting material had all been consumed. The reaction mixture was washed with 1N aqueous hydrochloric acid followed by a wash with

saturated aqueous sodium bicarbonate. The organic phase was separated and dried and the solvent removed therefrom in vacuo. The residue was dissolved in chloroform and chromatographed over Florisil using chloroform as the eluant. Fractions containing trans-(±)-1-cyano-6-oxo-decahydroquinoline were combined to yield 1.3 g. of a colorless oil (72.2%). The oil slowly crystallized. The crystals melted at 82-84°C.

Analysis Calculated: C, 67.39; H, 7.92; N, 15.72
Found: C, 67.29; H, 7.81; N, 15.42

The same reaction was carried out on 5.0 g. of trans-(-)-1-n-propyl-6-oxodecahydroquinoline in 150 ml. of methylene dichloride. Six and eight tenths grams of cyanogen bromide were added and the mixture stirred for 18 hours at room temperature under a nitrogen atmosphere. The reaction was worked up as above to yield a light yellow solid residue which was chromatographed over Florisil and eluted with chloroform as before. A colorless solid was obtained which melted at 119-120°C.; yield = 1.66 g.; $[\alpha]_D^{20°}$ (methanol) = -9.6°.

Analysis Calculated: C, 67.39; H, 7.92; N, 15.72;
Found: C, 67.67; H, 7.68; N, 15.87.

One and two-tenths grams of trans-(-)-1-cyano-6-oxodecahydroquinoline were dissolved in 50 ml. of 6N aqueous hydrochloric acid and the solution heated to reflux temperature for about 4 hours. The volatile constituents were removed in vacuo, the resulting residue was dissolved in water and the aqueous solution made basic with $NH_4OH$. The alkaline mixture was extracted several times with equal volumes of a 3:1

chloroform/isopropanol solvent mixture. The extracts were combined, washed with saturated aqueous sodium chloride and dried. Evaporation of the solvent in vacuo yielded 0.89 g. of an off-white solid (86.8% yield) comprising trans-(-)-6-oxodecahydroquinoline formed in the above reaction which compound melted at about 80°C. after crystallization from hexane. Trans-(-)-6-oxodecahydroquinoline thus prepared had the following characteristics.

Rotation $[\alpha]_D^{20°}$ = -33.2°

Mass spectrum: molecular ion at 153

Analysis Calculated: C, 70.55; H, 9.87; N, 9.14;

Found: C, 70.51; H, 9.61; N, 9.04.

## Example 3

Preparation of trans-($\pm$)-6-oxodecahydroquinoline from the 1-cyano derivative

A reaction mixture was prepared by dissolving 32.5 g of trans-($\pm$)-1-cyano-6-oxodecahydroquinoline in about 500 ml of 6 N hydrochloric acid and heating to reflux for three hours. The mixture was cooled to room temperature and allowed to stand for 18 hours. The volatile constituents were removed in vacuo and the resulting residue dissolved in water and made basic with ammonium hydroxide. The aqueous layer was extracted with several equal volume portions of a 3:1 chloroform/isopropanol solvent mixture. The organic extracts were combined, the combined extracts washed with saturated

aqueous sodium chloride and the solvent removed in vacuo to yield 25.6 g (91.6%) of a light yellow solid consisting of trans-(±)-6-oxodecahydroquinoline. Mass spectra peaks (m/e) = 153, 95 and 81. IR (KBr) is 3234 and 1712 $cm^{-1}$. NMR ($CDCl_3$) is δ 3.10 (bd, 1H) and δ 2.82 (m, 14H).

## Example 4

Preparation of trans-(±)-6-hydroxydecahydroquinoline from the 6-oxo derivative

A solution was prepared by dissolving 1.0 g of trans-(±)-6-oxodecahydroquinoline in 20 ml of methanol. The solution was cooled to 0°C and 0.66 g of sodium borohydride was added in portions. The mixture was stirred for one hour at room temperature, treated with 10 ml of saturated aqueous ammonium chloride, then diluted with water. The aqueous layer was made strongly basic with ammonium hydroxide, extracted with several equal volume portions of a 3:1 chloroform/isopropanol solvent mixture. The organic extracts were combined and the solvents evaporated to yield 0.86 g (86%) of a pale yellow solid consisting of trans-(±)-6-hydroxydecahydroquinoline. Mass spectra peaks (m/e) = 155, 136, 96, 82, and 68. IR (KBr) is 3400, 3268 and 3090 $cm^{-1}$.

## Example 5

Preparation of trans-(±)-6-oxodecahydroquinoline from the 6-hydroxy derivative

A reaction mixture was prepared by dissolving 0.7 g of trans-(±)-6-hydroxydecahydroquinoline in 3 ml of acetic acid. To the mixture was added 0.1 ml of concentrated sulfuric acid, then 0.5 ml of Jones Reagent (chromic anhydride in dilute sulfuric acid) was added dropwise with vigorous stirring. The mixture was exothermic and stirring was continued for 20 minutes at room temperature. To the mixture was added 1 ml of isopropanol and the flask was flushed with nitrogen. Water (5 ml), 1 g of trisodium citrate and 0.1 g of amalgamated zinc were added to the mixture with stirring for 20 minutes. The mixture was made strongly basic with 50% sodium hydroxide and was extracted with several equal volume portions of 3:1 chloroform/isopropanol solvent mixture. The organic extracts were combined, the combined extracts washed with saturated aqueous sodium chloride, dried over sodium sulfate, and evaporated in vacuo to yield a light yellow, waxy solid. The solid was sublimed to yield 0.1 g of colorless needles. The residue from the sublimation apparatus is chromatographed on a flask $SiO_2$ column with 4:1 methylene dichloride/10% methanol as eluant to yield an additional 0.21 g. Total yield 0.31 g (43.5%) of trans-(±)-6-oxodecahydroquinoline. IR and NMR are identical as given in Example 3.

## Example 6

Preparation of trans-(±)-6-oxodecahydroquinoline from the 6-ethyleneketal derivative

Ethyleneketal of trans-(±)-6-oxodecahydroquinoline, 0.39 g, was dissolved in 10 ml of 10% hydrochloric acid and stirred for one hour at room temperature. The reaction mixture was made basic with ammonium hydroxide. The aqueous layer was extracted with several equal volume portions of a 3:1 chloroform/isopropanol solvent mixture. The organic extracts were combined and evaporated in vivo to yield 0.17 g (56.5%) of a colorless crystalline residue consisting of trans-(±)-6-oxodecahydroquinoline. IR and NMR are identical as given in Example 3.

Compounds of formulae V and VI, trans-(±)- and trans-(-)-6-oxodecahydroquinoline are transformed to N-$C_1$-$C_3$ alkyl, allyl or benzyl derivatives by treatment with a $C_1$-$C_3$ alkyl halide, allyl halide or benzyl halide in a solvent such as acetone in the presence of base; for example, solid sodium carbonate. Sixty-seventy percent or higher yields of the desired 1-alkyl, allyl or benzyl derivative are obtained.

This reaction is particularly advantageous in preparing trans-(±)- and trans-(-)-2-amino(2-alkylamino or 2-dialkylamino)-6-allyl-5,5a,6,7,8,9,9a,10-octahydropyrimido[4,5-g]quinoline--discussed in our copending application 84305684.7.

## CLAIMS

1. A trans-($\pm$)-racemate of the formula

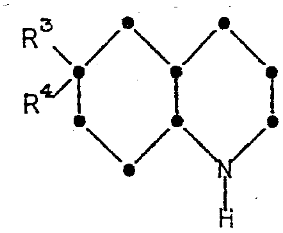

(VII)

wherein, when taken singly, one of $R^3$ and $R^4$ is H and the other OH, and when taken together, an oxo group, or a pharmaceutically-acceptable acid addition salt thereof.

2. A compound of claim 1 of the formula

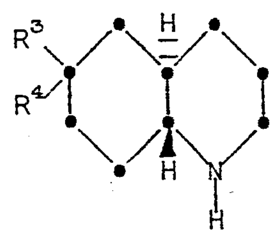

(VIII)

wherein, when taken singly, one of $R^3$ and $R^4$ is H and the other OH, and when taken together, an oxo group, or a pharmaceutically-acceptable acid addition salt thereof.

3. A compound of claim 1 or 2 wherein the pharmaceutically-acceptable acid addition salt is the hydrochloride salt.

4. A compound of claim 1 or 2 wherein $R^3$ and $R^4$ are taken together to form an oxo group.

5. A compound of claim 1 or 2 wherein one of $R^3$ and $R^4$ is H and the other OH.

6. Trans-(ℓ)-6-hydroxydecahydroquinoline.

7. Trans-(ℓ)-6-oxodecahydroquinoline.

8. Trans-(±)-6-hydroxydecahydroquinoline.

9. Trans-(±)-6-oxodecahydroquinoline.

10. A process for preparing a compound of formula (VII) or (VIII) as claimed in claim 1 or 2, which comprises:

(a) cleaving the N-cyano group from the corresponding starting compound by conventional methods; or

(b) oxidizing a compound of formula (VII) or (VIII) as claimed in claim 1 or 2 in which one of $R^3$ and $R^4$ is H and the other is OH with an oxidizing agent to provide the compounds of formula (VII) or (VIII) as claimed in claim 1 or 2 in which $R^3$ and $R^4$ are taken together to form an oxo group; or

(c) reducing a compound of formula (VII) or (VIII) as claimed in claim 1 or 2 in which $R^3$ and $R^4$ are taken together to form an oxo group with a reducing agent to provide the compounds of formula (VII) or (VIII) as defined above in which one of $R^3$ and $R^4$ is H and the other OH; or

(d) cleaving a trans-(±)-6-ketal compound of the formula

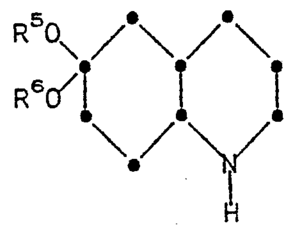

(XIII)

or its trans-(-)-enantiomer, wherein $R^5$ and $R^6$ are each $C_1$-$C_4$ alkyl or when taken together are $-CH_2-C(CH_3)_2-CH_2-$ or $-(CH_2)_n-$ where n is 2, 3 or 4, with formic acid or an aqueous acid to provide the compounds of formula (VII) or (VIII) as defined before in which $R^3$ and $R^4$ are taken together to form an oxo group; and

optionally followed by resolving a compound of formula (VII) to provide the corresponding trans-(-)-enantiomer; and

optionally salifying a compound of formula (VII) or (VIII) by conventional methods.